# EUROPÄISCHE PATENTANMELDUNG

(11) **EP 0 718 262 A1**
(43) Veröffentlichungstag der Anmeldung: **26.06.1996**
(21) Anmeldenummer: 95120170.6
(22) Anmeldetag: 20.12.1995
(51) Int. Cl.: C07C 17/278, C07C 19/16

(54) **Verfahren zur Herstellung von Perfluoralkyliodid-Telomeren**

(30) Priorität: 24.12.1994 DE 4446759
(71) Anmelder: HOECHST AKTIENGESELLSCHAFT, D-65926 Frankfurt am Main (DE)
(72) Erfinder: Werner, Konrad von, Dr., D-84518 Garching (DE)

(57) **Zusammenfassung**

Mittelkettige Perfluoralkyliodide werden in guter Selektivität durch Umsetzung eines kurzkettigen Perfluoralkyliodids mit Tetrafluorethylen erhalten, wenn die Telomerisationsreaktion mit Zink, Mangan, Vanadium, Rhenium, Rhodium, Ruthenium, Platin oder Silber katalysiert wird.

## Beschreibung

Mittel- bis längerkettige Perfluoralkyliodide sind Ausgangsmaterialien für die Herstellung von Fluortensiden sowie von hydro- und oleophobierenden Ausrüstungsmaterialien, beispielsweise für Textilien. Andererseits ist insbesondere das n-Perfluoroctyliodid ein Ausgangsmaterial für das n-Perfluoroctylbromid, das im medizinischen Bereich eine erhebliche Bedeutung erlangt hat.

Perfluoralkyliodide werden technisch durch Telomerisation entsprechend der Gleichung

RfI + n F₂C=CF₂ → Rf(CF₂-CF₂)ₙI ,

wobei Rf für Perfluoralkyl mit 1 bis 6 Kohlenstoffatomen und n für eine Zahl von 1 bis etwa 8 steht, hergestellt. Die Reaktion wird entweder durch Erhitzen (beispielsweise US-A 5 268 516) oder durch Radikalbildner (beispielsweise GB-A 1 535 408 oder US-A 5 068 471) gestartet. Bei der thermischen Reaktion bilden sich in erheblichem Ausmaß Perfluoralkane durch Dimerisierung der intermediär gebildeten Perfluoralkylradikale, und bei den radikalischen Reaktionen treten als Nebenprodukte wasserstoffhaltige Verbindungen auf.

Wie sich aus der obengenannten Reaktionsgleichung ergibt, wird die Bildung der im allgemeinen unerwünschten längerkettigen Telomeren durch eine hohe Konzentration des Telogens RfI zurückgedrängt. Eine Selektivität in Richtung auf erwünschte mittelkettige Telomere wird damit durch geringe Umsätze erkauft, womit ein hoher Destillationsaufwand an zurückzuführendem Telogen und niederkettigen Telomeren verbunden ist.

In einer als "Preliminary Note" gekennzeichneten Publikation beschreiben Chen et al., Journal of Fluorine Chemistry 36 (1987), Seiten 483 bis 489, den Einsatz von Kupfer als Telomerisationskatalysator. Dieses Verfahren hat den Vorteil, daß die Reaktion bereits bei 80 bis 100 °C abläuft und im Verhältnis zu Hochtemperatur-Telomerisationen kürzere Reaktionszeiten erfordert. Bei der Umsetzung von Perfluorethyliodid und Tetrafluorethylen im Molverhältnis von 1 : 2 bis 2 : 1 werden noch relativ große Mengen an unerwünschten längerkettigen Telomeren gebildet.

Es wurde nun gefunden, daß andere Metalle als Katalysatoren die Selektivität der Telomerisationsreaktion in Richtung auf die erwünschten mittelkettigen Produkte erhöhen, ohne daß dies durch geringere Umsätze erkauft werden muß. Da quantitative Vergleiche mit Metallkatalysatoren schwierig sind - es müßte immer die gleiche wirksame Oberfläche gewährleistet sein - gilt zumindest für die bevorzugten Ausführungsformen der Erfindung, daß auch bei erhöhten Umsätzen noch die Selektivität verbessert werden konnte.

Die Erfindung betrifft ein Verfahren zur Herstellung von Perfluoralkyliodid-Telomeren der Formel

Rf(CF₂-CF₂)ₙI ,

in der Rf für Perfluoralkyl mit 1 bis 6 Kohlenstoffatomen und n für eine Zahl von 1 bis etwa 4 steht, wobei das Maximum der Kohlenstoffatome im erhaltenen Telomerengemisch im Bereich von 6 bis 10 liegt, durch Umsetzung eines Perfluoralkyliodids der Formel RfI, in der Rf die vorstehend genannte Bedeutung hat, mit Tetrafluorethylen, das dadurch gekennzeichnet ist, daß als Katalysator Zink, Mangan, Vanadium, Rhenium, Rhodium, Ruthenium, Platin oder Silber eingesetzt wird. Bevorzugte Ausführungsformen der Erfindung werden im folgenden näher erläutert:

Die als Katalysator dienenden Metalle können in feinverteilter Form als Pulver oder auf einem inerten oder allenfalls schwachaktiven Träger eingesetzt werden. Als inerte oder schwachaktive Träger können auch andere Metalle wie Eisen, Kobalt, Nickel, Chrom, Molybdän, Wolfram, Titan oder deren Mischungen dienen. So können Autoklaven, deren Reaktionsraum mit einem solchen Metall ausgekleidet ist, mit einem katalytisch aktiven Metall überzogen werden.

Erfindungsgemäß können natürlich auch Mischungen der genannten katalytisch aktiven Metalle Einsatz finden. Von Vorteil sind einerseits Preiswerte aktive Metalle wie Zink oder Mangan. Allerdings müssen sie in vergleichsweise großen Mengen eingesetzt werden und führen auch zu einer gewissen Menge an Perfluoralkanen [durch Dimerisierung von Radikalen Rf(CF₂-CF₂)n·]. Bevorzugt sind deshalb Edelmetalle wie das relativ Preiswerte und aktive Silber, die diese Nachteile nicht zeigen und eine längere Standzeit aufweisen.

Als Telogene werden bevorzugt 2-Iodperfluorpropan, 1-Iodperfluorethan, -butan und -hexan. Es wurde gefunden, daß die Reaktionsgeschwindigkeit beim Einsatz von 1-Iodperfluorbutan und -hexan etwa um den Faktor 1,6 höher liegt als beim 1-Iodperfluorethan. Eine bevorzugte Ausführungsform der Erfindung besteht also darin, diese niederen Telomeren als Telogene - in reiner Form oder in Mischungen - einzusetzen.

Da die erfindungsgemäße Reaktion mehrphasig ist - das Gas Tetrafluorethylen wird mit dem flüssigen Telogen am festen Katalysator umgesetzt - ist für eine gute Durchmischung zu sorgen. Vorteilhaft sind deshalb Trägerkatalysatoren, bei denen das Metall auf einen Träger niedrigerer Dichte aufgebracht ist, da so die Flotierbarkeit in der flüssigen Perfluoralkyliodidphase verbessert wird. Weiterhin vorteilhaft ist der Einsatz eines Rührers, durch den das Tetrafluorethylen in die Flüssigphase eingegast wird. Günstig sind auch die sogenannten Strahlreaktoren, bei denen die Flüssigphase zusammen mit dem Katalysator über eine Düse umgepumpt wird. Das Gas - hier Tetrafluorethylen - wird kurz vor der Düse zudosiert. Die in der Düse erzeugten hohen Scherkräfte bewirken einen effektiven Gas-Flüssig-Übergang. Diese Reaktoren eignen sich auch für den kontinuierlichen Betrieb.

Eine vorteilhafte Ausführungsform der Erfindung besteht im Einsatz eines Rohrreaktors, beispielsweise aus Edelstahl, welcher mit einer Temperaturregelung für Heizung und Kühlung und einer Druckstandhaltung am Ausgang versehen ist. In diesen wird der Katalysator stationär eingebracht. Die Edukte, Perfluoralkyliodid und Tetrafluorethylen, werden am Eingang des Reaktors zudosiert. Je nach Telogen, zum Beispiel 1-Iod-perfluorethan oder 1-Iod-n-perfluorbutan, wird der Innendruck so gewählt, daß die Perfluoralkyliodide während der Reaktion überwiegend flüssig sind. Dies ist durch Einstellung von Drücken im Bereich von 8 bis 18 bar möglich.

Es wurde weiterhin gefunden, daß Wasser - bei Sauerstoffausschluß - nicht zu einer vermehrten Nebenproduktbildung führt und auch die Reaktion nur wenig beeinträchtigt. Es ist also nicht erforderlich, die Ausgangsmaterialien zu trocknen.

Weitere bevorzugte Ausführungsformen der Erfindung ergeben sich aus den folgenden Beispielen.

### Beispiele

Die Ergebnisse wurden gaschromatographisch ausgewertet, wobei mittels experimentell kalibrierter Flächenfaktoren eine direkte Auswertung der gefundenen Daten in Form von Gewichtsanteilen erhalten wurde.

Die eingesetzten Perfluoralkyliodide wurden vor Gebrauch mit Kaliumcarbonat-Pulver von Verunreinigungen wie Fluorwasserstoff oder Iod befreit und unter Stickstoff gelagert. Gaschromatographisch ermittelte Reinheit:

| | |
|---|---|
| F₂C=CF₂ | 99,98 % |
| n-C₂F₅I | 97,5 % |
| n-C₄F₉I | 99,8 % |
| n-C₆F₁₃I | 98,7 % |
| i-C₃F₇I | 99,5 % |

### Beispiele 1 bis 13

Die Umsetzungen wurden in einem 300-ml-Schüttelautoklav aus Edelstahl (V₄A, Material 1,4571) durchgeführt. Die angegebene Katalysatormenge wurde unter Stickstoff vorgelegt. Nach einer Druckprobe mit 50 bar Stickstoff wurde der Autoklav bis 1 mbar evakuiert, dann bis -78 °C abgekühlt. Nach Einsaugen der angegebenen Perfluoralkyliodid-Menge wurde bei -78 °C die gewünschte Menge an Tetrafluorethylen (in den Tabellen "TFE") einkondensiert (die Wägung muß rasch erfolgen, um Fehler durch Vereisung des Autoklaven zu vermeiden).

### Beispiele 1 bis 3 und Vergleichsbeispiel V1

50 g 1-Iodpentafluorethan wurden mit der angegebenen Katalysatormenge im angegebenen Molverhältnis mit Tetrafluorethylen 5 Stunden bei 100 °C geschüttelt. Die Ergebnisse zeigt die Tabelle 1. In der letzten Spalte ist unter "Σ X" die Summe aller Nicht-RfI-Verbindungen zusammengefaßt. Sie wurden bei der gaschromatographischen Auswertung pauschal mit dem Massefaktor 1,00 gewichtet. Es handelt sich beispielsweise um Tetrafluorethylen, RfH-Verbindungen (die in geringer Menge in den Edukten enthalten sind), Perfluoralkane und Wasser (das beim Dosieren der Proben mit einer gekühlten Spritze eingeschleppt wurde).

**Tabelle 1**

| Beispiel | Katalysator | | | Molverhältnis C₂F₅I/TFE | Produktverteilung C₂F₅(CF₂-CF₂)ₙ-I [Masse-%], n = | | | | | | | | | | | |
|---|---|---|---|---|---|---|---|---|---|---|---|---|---|---|---|---|
| | | [g] | [mmol] | | 0 | 1 | 2 | 3 | 4 | 5 | 6 | 7 | 8 | 9 | 10 | Σ X |
| V1 | Cu | 2,0 | 31,5 | 2,22 | 63,4 | 11,9 | 7,2 | 5,3 | 3,9 | 2,9 | 1,9 | 1,0 | 0,4 | --- | --- | 2,1 |
| 1 | V | 1,0 | 19,6 | 2,22 | 60,4 | 12,8 | 8,3 | 5,8 | 3,9 | 2,6 | 1,7 | 1,0 | 0,5 | 0,2 | 0,1 | 2,7 |
| 2 | Mn | 2,0 | 36,4 | 2,22 | 65,1 | 12,2 | 7,3 | 4,7 | 3,1 | 1,9 | 1,2 | 0,7 | 0,4 | 0,1 | --- | 3,3 |
| 3 | Rh | 0,1 | 0,97 | 2,0 | 74,8 | 7,3 | 4,9 | 3,6 | 2,6 | 1,7 | 1,1 | 0,6 | 0,2 | 0,1 | --- | 3,1 |

### Beispiele 4 bis 7 und Vergleichsbeispiel V2

Analog den vorhergehenden Beispielen wurden hier 69,2 g 1-Iod-n-perfluorbutan eingesetzt und der Ansatz wieder 5 Stunden bei 100 °C geschüttelt. Im Beispiel 6 enthielt der Katalysator 0,5 ml Wasser. Im Beispiel 7 wurde das Silber als Trägerkatalysator mit 10 Gew.-% Silber auf Aluminiumoxid eingesetzt.

**Tabelle 2**

| Beispiel | Katalysator | | | Molverhältnis C₄F₉I/TFE | Produktverteilung C₂F₅(CF₂-CF₂)ₙ-I [Masse-%], n = | | | | | | | | | | |
|---|---|---|---|---|---|---|---|---|---|---|---|---|---|---|---|
| | | [g] | [mmol] | | 1 | 2 | 3 | 4 | 5 | 6 | 7 | 8 | 9 | 10 | Σ X |
| V2 | Cu | 2,0 | 31,5 | 1,82 | 54,5 | 25,2 | 11,1 | 4,7 | 1,9 | 0,8 | 0,4 | 0,2 | 0,1 | < 0,1 | 1,1 |
| 4 | Mn | 2,0 | 36,5 | 1,74 | 52,2 | 23,4 | 10,8 | 5,1 | 2,3 | 1,1 | 0,5 | 0,3 | 0,1 | < 0,1 | 4,1 |
| 5 | Mn | 2,0 | 36,5 | 1,74 | 55,7 | 21,9 | 10,3 | 4,7 | 2,2 | 1,1 | 0,6 | 0,3 | 0,2 | 0,1 | 3,0 |
| 6 | V | 1,0 | 19,6 | 2,11 | 63,0 | 20,4 | 7,8 | 2,9 | 1,2 | 0,5 | 0,3 | 0,2 | 0,1 | --- | 3,2 |
| 7 | Ag | 0,5 | 4,6 | 1,87 | 65,4 | 20,9 | 8,1 | 3,0 | 1,1 | 0,4 | 0,1 | 0,1 | --- | --- | 0,9 |

### Beispiele 8 bis 11

69,2 g 1-Iod-n-perfluorbutan wurden mit 2,0 g Zink (30,6 mmol) mit Tetrafluorethylen im angegebenen Molverhältnis bei unterschiedlichen Temperaturen 5 Stunden geschüttelt. Die näheren Angaben und die Ergebnisse zeigt die Tabelle 3.

**Tabelle 3**

| Beispiel | Molverhältnis C₄F₉I/TFE | Temperatur [°C] | Produktverteilung C₂F₅(CF₂-CF₂)ₙ-I [Masse-%], n = | | | | | | | | | |
|---|---|---|---|---|---|---|---|---|---|---|---|---|
| | | | 1 | 2 | 3 | 4 | 5 | 6 | 7 | 8 | 9 | Σ X |
| 8 | 1,94 | 100 | 52,6 | 23,0 | 9,7 | 3,8 | 1,4 | 0,5 | 0,2 | 0,1 | --- | 8,7 |
| 9 | 1,87 | 90 | 48,7 | 24,8 | 12,3 | 6,7 | 3,4 | 1,4 | 0,6 | 0,2 | 0,1 | 1,8 |
| 10 | 2,0 | 60 | 51,0 | 25,8 | 11,7 | 5,6 | 2,6 | 1,0 | 0,4 | 0,2 | 0,1 | 1,6 |
| 11 | 2,06 | 40 | 92,4 | 2,4 | 0,9 | 0,5 | 0,3 | 0,2 | 0,1 | --- | --- | 3,2 |

### Beispiele 12 und 13

98,5 g 1-Iod-n-perfluorhexan wurden mit dem angegebenen Katalysator und Tetrafluorethylen im angegebenen Molverhältnis 5 Stunden bei 100 °C geschüttelt. Die näheren Angaben und die Produktverteilung zeigt die Tabelle 4.

Zur Verbesserung der Übersichtlichkeit wurde in die Tabelle 4 auch das folgende Beispiel aufgenommen.

### Beispiel 14

Analog Beispiel 13 wurden die gleichen Reaktanten in einem Glaskolben umgesetzt, der mit Rührer, Thermometer und Kühler mit einem auf 130 mbar Überdruck eingestellten Ventil am Kopfende ausgerüstet war. Das Tetrafluorethylen wurde innerhalb von 3 Stunden in den Kolben eingeleitet. Die gesamte Reaktionsdauer betrug 5 Stunden, die Temperatur 110 °C.

**Tabelle 4**

| Beispiel | Katalysator | | | Molverhältnis C₆F₁₃I/TFE | Produktverteilung C₂F₅(CF₂-CF₂)ₙ-I [Masse-%], n = | | | | | | | | |
|---|---|---|---|---|---|---|---|---|---|---|---|---|---|
| | | [g] | [mmol] | | 2 | 3 | 4 | 5 | 6 | 7 | 8 | 9 | Σ X |
| 12 | Cu | 2,0 | 31,5 | 2,0 | 53,0 | 24,5 | 9,8 | 3,6 | 1,3 | 0,5 | 0,2 | 0,1 | 6,5 |
| 13 | Mn | 2,0 | 36,5 | 1,90 | 55,9 | 23,9 | 10,7 | 4,7 | 2,0 | 0,8 | 0,3 | 0,1 | 1,5 |
| 14 | Mn | 2,0 | 36,5 | 2,0 | 53,5 | 24,4 | 9,5 | 3,4 | 1,2 | 0,4 | 0,2 | 0,1 | 7,3 |

### Beispiele 15 bis 17

865 g 1-Iod-n-perfluorbutan wurden im Molverhältnis 2 : 1 mit Tetrafluorethylen in einem 1-l-Rührkessel mit den angegebenen Katalysatoren und bei der angegebenen Temperatur umgesetzt. Der Rührkessel war mit einer Hülse für das Thermoelement und einem Ankerrührer (maximale Drehzahl 350 Upm) ausgerüstet. Die Ergebnisse zeigt die Tabelle 5.

### Beispiele 18 bis 20

Die Umsetzung erfolgte analog den Beispielen 15 bis 17, jedoch mit einem Begasungsrührer, wobei das Tetrafluorethylen durch die hohle Rührerwelle zugeführt und über feine Bohrungen am Ende der Rührerblätter in Form feiner Bläschen in die Flüssigphase verwirbelt wurde. Der Begasungsrüher hatte eine maximale Drehzahl von 500 Upm. Die weiteren Angaben und Ergebnisse zeigt ebenfalls die Tabelle 5.

Die Reaktionszeit betrug 5 Stunden für die Beispiele 15 bis 17 und 2,5 Stunden für die Beispiele 18 bis 20.

**Tabelle 5**

| Beispiel | Katalysator | | | Temperatur [°C] | Produktverteilung C₂F₅(CF₂-CF₂)ₙ-I [Masse-%], n = | | | | | | | | |
|---|---|---|---|---|---|---|---|---|---|---|---|---|---|
| | | [g] | [mmol] | | 1 | 2 | 3 | 4 | 5 | 6 | 7 | 8 | Σ X |
| 15 | Zn | 25,0 | 382 | 90 bis 100 | 74,6 | 12,8 | 3,8 | 1,1 | 0,3 | 0,1 | | | 2,3 |
| 16 | Mn | 27,5 | 500 | 110 | 78,5 | 11,3 | 3,5 | 1,0 | 0,2 | 0,1 | | | 5,4 |
| 17 | Zn | 25,0 0,5 | 382 4,6 | 95 bis 100 | 63,4 | 18,9 | 6,7 | 2,2 | 0,7 | 0,3 | | | 2,2 |
| | + Ag | 0,5 | 4,6 | | | | | | | | | | |
| 18 | Zn | 15,0 | 229 | 90 | 51,1 | 28,5 | 12,5 | 4,4 | 1,3 | 0,3 | 0,1 | | 1,8 |
| 19 | Mn | 15,0 | 273 | 100 | 52,5 | 27,5 | 11,6 | 3,8 | 1,2 | 0,3 | 0,1 | | 3,0 |
| 20 | Zn + Ag | 10,0 0,2 | 153 1,8 | 90 | 50,7 | 28,8 | 12,8 | 4,0 | 1,2 | 0,4 | 0,2 | 0,1 | 1,8 |
| | + Ag | 0,2 | 1,8 | | | | | | | | | | |

### Beispiel 21

In dem für die Beispiele 1 bis 13 genannten Autoklav wurden 29,6 g (0,1 mol) Perfluorisopropyliodid und 5,0 g (0,05 mol) Tetrafluorethylen mit 1,0 g Zink (15,3 mmol) 3 Stunden bei 80 °C umgesetzt. Die Produktverteilung zeigt die Tabelle 6.

**Tabelle 6**

| Beispiel | Produktverteilung (CF₃)₂CF(CF₂-CF₂)ₙ-I [Masse-%], n = | | | | | | | | | | |
|---|---|---|---|---|---|---|---|---|---|---|---|
| | 0 | 1 | 2 | 3 | 4 | 5 | 6 | 7 | 8 | 9 | Σ X |
| 21 | 57,1 | 18,0 | 11,4 | 6,3 | 2,9 | 1,4 | 0,7 | 0,4 | 0,2 | 0,1 | 1,5 |

### Beispiele 22 bis 29

In einem 250-ml-Schüttelautoklav wurden zwei Versuchsreihen (A: Beispiele 22 bis 26 und B: Beispiele 27 bis 29) durchgeführt. Hierbei wurden folgende Katalysatoren getestet (Prozente sind Masse-%):
10 % Silber auf Al₂O₃
2 % Palladium auf Al₂O₃
1 % Platin auf Al₂O₃
2 % Rhenium auf Al₂O₃-SiO₂
5 % Ruthenium auf Al₂O₃
5 % Rhodium auf Aktivkohle
Es wurden jeweils 0,2 mol 1-Iod-n-perfluorbutan, 0,1 mol Tetrafluorethylen und 1 Mol-% Metall, bezogen auf das 1-Iod-n-perfluorbutan, eingesetzt und 5 Stunden bei 100 °C umgesetzt.

In Versuchsreihe A wurden die Katalysatoren in handelsüblicher Form ohne zusätzliche Aktivierung verwendet. In Versuchsreihe B wurden Silber und Rhenium nicht geprüft, weil sie ohnehin eine sehr hohe Aktivität besitzen. Die anderen Katalysatoren wurden auf folgende Weise aktiviert:

Der Katalysator (je 1 Mol-%, bezogen auf n-C₄F₉I) wurde in einem 250-ml-Autoklav mit je 10 bar H₂ und 10 bar N₂ während 5 Stunden bei 160 °C behandelt. Nach Entspannen wurde mit N₂ gespült und der Katalysator wurde noch 30 Minuten mit 20 bar N₂ behandelt, um adsorbierten Wasserstoff zu entfernen. Nach Abkühlen auf Raumtemperatur wurde evakuiert und die eigentliche Reaktion begonnen. Die Ergebnisse zeigt die Tabelle 7.

**Tabelle 7**

| Beispiel | Katalysator | Produktverteilung C₂F₅(CF₂-CF₂)ₙ-I [Masse-%], n = | | | | | | | | | |
|---|---|---|---|---|---|---|---|---|---|---|---|
| | | 1 | 2 | 3 | 4 | 5 | 6 | 7 | 8 | Σ Perfluoralkane | Σ X |
| Reihe A: | | | | | | | | | | | |
| 22 | Ag auf Al₂O₃ | 61,5 | 21,8 | 9,7 | 4,0 | 1,6 | 0,9 | 0,3 | --- | 0,19 | --- |
| 23 | Pt auf Al₂O₃ | 88,0 | 6,8 | 2,7 | 1,1 | 0,5 | 0,3 | 0,1 | --- | 0,05 | 0,4 |
| 24 | Ru auf Al₂O₃ | 85,3 | 8,9 | 3,3 | 1,3 | 0,5 | 0,3 | 0,2 | 0,1 | 0,06 | 0,1 |
| 25 | Rh auf Aktivkohle | 93,5 | 3,6 | 1,3 | 0,5 | 0,3 | 0,1 | --- | --- | 0,36 | 0,3 |
| 26 | Re auf Al₂O₃-SiO₂ | 58,3 | 23,6 | 10,6 | 4,4 | 1,8 | 0,8 | 0,3 | 0,1 | 0,07 | --- |

| Reihe B: | | | | | | | | | | | |
|---|---|---|---|---|---|---|---|---|---|---|---|
| 27 | Pt auf Al₂O₃ | 52,3 | 26,3 | 11,9 | 5,2 | 2,2 | 1,0 | 0,5 | 0,2 | 0,07 | 0,3 |
| 28 | Ru auf Al₂O₃ | 58,6 | 21,8 | 9,8 | 4,9 | 2,4 | 1,2 | 0,7 | 0,2 | 0,09 | 0,2 |
| 29 | Rh auf Aktivkohle | 77,8 | 14,7 | 4,6 | 1,4 | 0,5 | 0,3 | 0,1 | 0,1 | 0,07 | 0,4 |

## Patentansprüche

1. Verfahren zur Herstellung von Perfluoralkyliodid-Telomeren der Formel
Rf(CF₂-CF₂)ₙI ,
in der Rf für Perfluoralkyl mit 1 bis 6 Kohlenstoffatomen und n für eine Zahl von 1 bis etwa 4 steht, wobei das Maximum der Kohlenstoffatome im erhaltenen Telomerengemisch im Bereich von 6 bis 10 liegt, durch Umsetzung eines Perfluoralkyliodids der Formel RfI, in der Rf die vorstehend genannte Bedeutung hat, mit Tetrafluorethylen, dadurch gekennzeichnet, daß als Katalysator Zink, Mangan, Vanadium, Rhenium, Rhodium, Ruthenium, Platin oder Silber eingesetzt wird.

2. Verfahren nach Anspruch 1, dadurch gekennzeichnet, daß das katalytisch aktive Metall auf einem Träger fixiert ist.

3. Verfahren nach Anspruch 1 oder 2, dadurch gekennzeichnet, daß das Metall Silber ist.

4. Verfahren nach einem oder mehreren der vorgehenden Ansprüche, dadurch gekennzeichnet, daß das Tetrafluorethylen mit einem Rührer in die Flüssigphase eingegast wird.
